# EUROPÄISCHE PATENTANMELDUNG

(11) **EP 0 751 112 A2**
(43) Veröffentlichungstag der Anmeldung: **02.01.1997**
(21) Anmeldenummer: 96109474.5
(22) Anmeldetag: 13.06.1996
(51) Int. Cl.: C07C 49/597, C07C 49/603, C07C 49/607, C07C 45/61

(54) **Verfahren zur Herstellung von Arylidensubstituierten Alkylcycloalkanonen**

(30) Priorität: 28.06.1995 DE 19523450
(71) Anmelder: BAYER AG, 51368 Leverkusen (DE)
(72) Erfinder: Müller, Nikolaus, Dr., 40789 Monheim (DE); Essert, Thomas, Dr., 51491 Overath (DE)

(57) **Zusammenfassung**

Aryliden-substituierte Alkylcycloalkanone werden aus Alkyl-substituierten Cycloalkanonen durch Umsetzung mit aromatischen Carbonylverbindungen in Gegenwart eines basischen Katalysators und Wasser hergestellt.

## Beschreibung

Die vorliegende Erfindung betrifft ein besonders vorteilhaftes Verfahren zur Herstellung von Aryliden-substituierten Alkylcycloalkanonen durch Kondensation von Alkylcycloalkanonen mit aromatischen Aldehyden oder Aralkylketonen.

Es ist bekannt, daß man Spiro-[2,4]-heptan-4-on mit 4-Chlorbenzaldehyd in methanolischer Natriumhydroxidlösung zu 5-(4-Chlorbenzyliden)-spiro-[2,4]-heptan-4-on umsetzen kann (siehe DE-OS 43 20 498, Beispiel 1). Nachteilig ist die benötigte extrem lange Reaktionszeit von 50 Stunden.

Weiterhin ist bekannt 2-Methylcyclohexanon mit Benzaldehyd in Gegenwart einer Lösung von Natriumhydroxid in einem Gemisch aus 37,5 Vol-% Alkohol und 62,5 Vol-% Wasser umzusetzen. So kann im Verlauf von 3 Stunden in 65 bis 70 %iger Ausbeute 2-Benzal-6-methylcyclohexanon (= 2-Methyl-6-benzylidencyclohexanon) erhalten werden (siehe J. Am. Chem. Soc. 65, 1317 (1943)). Nachteilig ist die relativ geringe Ausbeute und das Anfallen des Produktes als Öl, das extrahiert und destillativ aufgearbeitet werden muß.

Es besteht deshalb noch ein Bedürfnis nach einem Verfahren, mit dem sich Aryliden-substituierte Alkylcycloalkanone mit hohen Ausbeuten, in kurzen Reaktionszeiten und ohne aufwendige Aufarbeitung herstellen lassen.

Es wurde nun ein Verfahren zur Herstellung von Aryliden-substituierten Alkylcycloalkanonen der Formel (I) 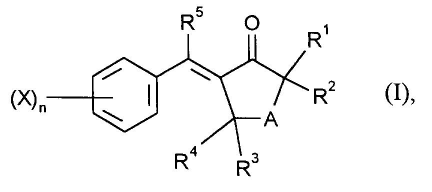 in der
- A: für gegebenenfalls substituiertes -(CH₂)-ₓ mit x = 1, 2 oder 3 steht,
- R¹ und R²: für gleiche oder verschiedene, gegebenenfalls substituierte C₁-C₄-Alkyl- oder C₃-C₇-Cycloalkylreste stehen oder R¹ und R² gemeinsam mit dem dazwischenliegenden C-Atom einen C₃-C₇-Cycloalkylrest bilden,
- R³, R⁴ und R⁵: gleich oder verschieden sind und für Wasserstoff oder gegebenenfalls substituiertes C₁-C₄-Alkyl oder C₃-C₇-Cycloalkyl stehen, wobei R³ und R⁴ gemeinsam mit dem dazwischenliegenden C-Atom auch einen C₃-C₇-Cycloalkylrest bilden können,
- X: für Halogen, Cyano, Nitro, C₁-C₄-Alkyl, C₁-C₄-Halogenalkyl, C₁-C₄-Alkoxy oder C₁-C₄-Halogenalkoxy stehen, wobei beim Vorhandensein mehrerer X-Reste diese gleich oder verschieden sein können
und
- n: für null oder eine ganze Zahl von 1 bis 5 steht,
gefunden, das dadurch gekennzeichnet ist, daß man substituierte Cycloalkanone der Formel (II) 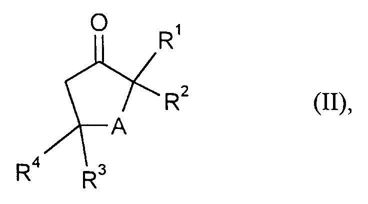 in der
die verwendeten Symbole die bei Formel (I) angegebene Bedeutung haben, mit aromatischen Carbonylverbindungen der Formel (III) 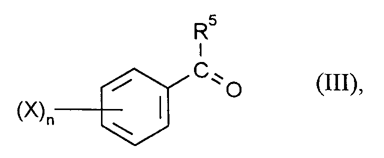 in der
die verwendeten Symbole die bei Formel (I) angegebene Bedeutung haben,
in Gegenwart eines basischen Katalysators und Wasser umsetzt.

A kann gegebenenfalls einen oder mehrere, gleiche oder verschiedene Substituenten aus der Gruppe C₁-C₄-Alkyl und C₃-C₇-Cycloalkyl enthalten, wobei C₁-C₄-Alkyl seinerseits gegebenenfalls durch Halogen oder C₁-C₄-Alkoxy und C₃-C₇-Cycloalkyl seinerseits gegebenenfalls durch Halogen, C₁-C₄-Alkyl oder C₁-C₄-Alkoxy substituiert sein kann. Vorzugsweise steht A für -CH₂-, -CH₂-CH₂- oder - CH₂-CH₂-CH₂-, d.h. vorzugsweise werden 2-Alkyl- oder 2,2-Dialkyl-cyclopentanone, -cyclohexanone oder -cycloheptanone der Formel (II) mit substituierten Benzaldehyden der Formel (III) zu 2-Alkyl- oder zu 2,2-Dialkyl-5-aryliden-cyclopentanonen, zu 2-Alkyl oder zu 2,2-Dialkyl-6-aryliden-cyclohexanonen oder zu 2-Alkyl- oder zu 2,2-Dialkyl-7-aryliden-cycloheptanonen der Formel (I) umgesetzt.

Soweit R¹ und R² für C₁-C₄-Alkyl stehen, kann dieses gegebenenfalls durch Halogen, C₁-C₄-Alkoxy, Halogen-C₁-C₄-alkoxy, C₂-C₄-Alkenyl, C₂-C₄-Alkinyl, Halogen-C₂-C₄-alkenyl oder Halogen-C₂-C₄-alkinyl, substituiert sein. Soweit R¹ und R² für C₃-C₇-Cycloalkyl steht, kann dies durch Halogen, C₁-C₄-Alkoxy oder C₁-C₄-Alkyl substituiert sein. Vorzugsweise stehen R¹ und R² unabhängig voneinander für unsubstituiertes C₁-C₄-Alkyl oder unsubstituiertes C₃-C₄-Alkenyl.

Soweit R³, R⁴ und R⁵ für C₁-C₄-Alkyl stehen, kann dieses gegebenenfalls durch Halogen oder C₁-C₄-Alkoxy substituiert sein. Soweit R³ und R⁴ für C₃-C₇-Cycloalkyl stehen, kann dieses gegebenenfalls durch Halogen, C₁-C₄-Alkyl oder C₁-C₄-Alkoxy substituiert sein. Vorzugsweise stehen R³, R⁴ und R⁵ für Wasserstoff.
- X: steht vorzugsweise für Fluor, Chlor, Cyano oder C₁-C₄-Alkyl.
- n: steht vorzugsweise für null, 1 oder 2. Im Falle n = 1 befindet sich X vorzugsweise in 4-Stellung. Im Falle n = 2 befindet sich X vorzugsweise in 2- und 4-Stellung.

Besonders bevorzugte Ausgangsmaterialien der Formel (II) sind 2-Methylcyclopentanon, 2,2-Dimethylcyclopentanon, 2-Ethylcyclopentanon, 2,2-Diethylcyclopentanon, 2-Isopropylcyclopentanon, 2-Methylcyclohexanon und 2-Methylcycloheptanon.

Besonders bevorzugte Verbindungen der Formel (III) sind: 4-Chlorbenzaldehyd, 4-Fluorbenzaldehyd, 4-Methoxybenzaldehyd, 4-Methylbenzaldehyd und 2,4-Dichlorbenzaldehyd.

Als basische Katalysatoren können z.B. Alkali- und Erdalkalihydroxide wie Lithiumhydroxid, Natriumhydroxid, Kaliumhydroxid, Cäsiumhydroxid, Magnesiumhydroxid, Calciumhydroxid, Strontiumhydroxid und Bariumhydroxid und Alkali- und Erdalkalicarbonate wie Natriumcarbonat, Kaliumcarbonat, Calciumcarbonat und Magnesiumcarbonat und Oxide der genannten Elemente eingesetzt werden, die unter den Reaktionsbedingungen Hydroxide oder Carbonate bilden.

Bevorzugte basische Katalysatoren sind Natriumhydroxid, Kaliumhydroxid, Magnesiumhydroxid, Calciumhydroxid, Natriumcarbonat und Kaliumcarbonat. Besonders bevorzugt sind Natriumhydroxid und Kaliumhydroxid.

Es ist ein wesentliches Merkmal der vorliegenden Erfindung, daß als Reaktionsmedium Wasser eingesetzt wird. Das Reaktionsmedium kann gegebenenfalls bis zu 20 Vol-%, vorzugsweise bis zu 10 Vol-% und insbesondere bis zu 5 Vol-% organische Lösungsmittel enthalten, z.B. Alkohole wie Methanol, Ethanol, n-Propanol, i-Propanol und Butanole, Sulfone wie Dimethylsulfoxid und Tetramethylensulfon, Ether wie Tetrahydrofuran und Amide wie Dimethyl-, Diethyl-, Di-n- und -i-propyl-, Dibutyl-, Dipentyl-, Dihexyl-, Dicyclohexylformamid, N-Methylpyrrolidon, N-Methylpiperidon, N-Methylcaprolactam, höhere N-Alkylpyrrolidine, -piperidone und -caprolactame.

Ganz besonders bevorzugt wird Wasser ohne Zusätze anderer Lösungsmittel eingesetzt.

Das erfindungsgemäße Verfahren kann z.B. bei Temperaturen von 20 bis 200°C durchgeführt werden. Bevorzugt sind Temperaturen von 50 bis 170°C, insbesondere solche von 90 bis 150°C. Bei Reaktionstemperaturen oberhalb des Siedepunktes des Reaktionsgemisches muß im geschlossenen Gefäß bei dem sich selbst einstellenden Druck oder bei Drucken von z.B. bis zu 10 bar gearbeitet werden.

Die Menge des einzusetzenden Reaktionsmediums kann z.B. zwischen 100 und 1000 ml pro Mol eingesetzter Verbindung der Formel (II) betragen. Vorzugsweise beträgt diese Menge 300 bis 700 ml, besonders bevorzugt 400 bis 600 ml.

Die Ausgangsmaterialien der Formeln (II) und (III) werden bevorzugt im äquimolaren Verhältnis eingesetzt. Überschüsse der einen oder anderen Komponente sind auch möglich, z.B. 0,5 bis 2 Mol der Verbindung der Formel (II) pro Mol der Verbindung der Formel (III).

Das Mengenverhältnis von Katalysator zu Verbindung der Formel (II) kann z.B. von 0,02 bis 0,2 Äquivalente pro Mol betragen. Bevorzugt sind 0,05 bis 0,1, insbesondere 0,07 bis 0,09 Äquivalente Katalysator pro Mol der Verbindung der Formel (II).

Die Aufarbeitung des nach Durchführung des erfindungsgemäßen Verfahrens vorliegenden Reaktionsgemisches kann auf sehr einfache Weise erfolgen, z.B. indem man es abkühlt, z.B. auf Raumtemperatur, und den vorliegenden Festkörper abfiltriert, mit Wasser wäscht und trocknet.

Eine bevorzugte Ausführungsform des erfindungsgemäßen Verfahrens wird im folgenden beispielhaft an der Umsetzung von 2-Methylcyclopentanon mit 4-Chlorbenzaldehyd in Gegenwart von Kaliumhydroxid erläutert:
Eine Lösung aus Kaliumhydroxid und Wasser wird im Reaktionsgefäß, z.B. einem Druckautoklaven, vorgelegt und 2-Methylcyclopentanon und 4-Chlorbenzaldehyd zugegeben. Man erhitzt auf Reaktionstemperatur unter Eigendruck (bei Reaktionstemperaturen unter 100°C kann auch drucklos gearbeitet werden) und läßt eine gewisse Zeit bei dieser Temperatur nachreagieren. Nach Abkühlung auf Raumtemperatur wird das auskristallisierte Produkt abgesaugt, mit Wasser gewaschen und getrocknet.

Es ist ausgesprochen überraschend, daß die erfindungsgemäße Aldolkondensation im wäßrigen Medium mit in Wasser schwer löslichen Verbindungen der Formel (III) so gut durchführbar ist. Die erzielbaren Ausbeuten liegen im allgemeinen über 85 % der Theorie, häufig über 90 % der Theorie bei kurzen Reaktionszeiten.

Es ist weiterhin überraschend, daß trotz des Einsatzes von stark alkalischen Katalysatoren keine nennenswerten Ausbeuteverluste durch Cannizzaro-Reaktionen auftreten. Das erfindungsgemäße Verfahren ermöglicht die Herstellung von Aryliden-substituierten Alkyl-cycloalkanonen in hohen Ausbeuten und ist sehr einfach durchführbar.

Ein besonderer Vorteil des erfindungsgemäßen Verfahrens liegt in der Verwendung von Wasser als Reaktionsmedium, da aufwendige Lösungsmittelrückführungen dann nicht notwendig sind. Die gewünschten Reaktionsprodukte der Formel (I) können auf einfache Art und Weise abgetrennt und isoliert werden. Die Möglichkeit bei höheren Temperaturen zu arbeiten führt zu kürzeren Reaktionszeiten, was weniger Nebenprodukte zur Folge hat. Die Reaktionszeit beträgt häufig weniger als 14 Stunden, insbesondere 2 bis 11 Stunden.

Die nach dem erfindungsgemäßen Verfahren herstellbaren Aryliden-substituierten Alkylcycloalkanone der Formel (I) sind wertvolle Zwischenprodukte für die Herstellung von Pestiziden, speziell Fungiziden (siehe EP-OS 378 953, EP-OS 537 909 und EP-OS 329 397).

### Beispiele

Prozentangaben sind, soweit nichts anderes vermerkt ist, Gewichtsprozent.

### Beispiel 1

In einem 3 l Edelstahl-Autoklaven wurde eine Lösung aus 16,8 g 85 %iger wäßriger Kalilauge und 1500 ml Wasser vorgelegt, 294 g 2-Methylcyclopentanon und 421,8 g 4-Chlorbenzaldehyd zugesetzt und dann unter Eigendruck auf 120°C erhitzt. Bei dieser Temperatur wurde 3 Stunden nachgerührt. Nach Abkühlung auf Raumtemperatur wurde das feinkörnige, gelbe Produkt abgesaugt und mit Wasser neutral gewaschen und anschließend im Vakuumtrockenschrank bei 60°C getrocknet. So wurden 658,5 g 2-Methyl-5-(4-chlorbenzyliden)-cyclopentanon (93,1 %ig) erhalten. Das entspricht einer Ausbeute von 93 % der Theorie.

### Beispiel 2

In einer 3-Halskolbenrührapparatur mit Rückflußkühler wurde eine Lösung aus 2,8 g 85 %iger wäßriger Kalilauge und 250 ml Wasser vorgelegt, 68 g p-Anisaldehyd und 49 g 2-Methylcyclopentanon zugegeben und 3 Stunden unter Rückfluß gekocht. Anschließend wurde auf Raumtemperatur abgekühlt und der auskristallisierte gelbe Feststoff abgesaugt, mit Wasser neutral gewaschen und im Vakuumtrockenschrank bei 60°C getrocknet. So wurden 98,3 g 2-Methyl-5-(4-methoxybenzyliden)-cyclopentanon (93,2 %ig) erhalten. Das entspricht einer Ausbeute von 85 % der Theorie.

### Beispiel 3

In einem 0,7 l Edelstahl-Autoklaven wurde eine Lösung aus 2,8 g 85 %iger wäßriger Kalilauge und 250 ml Wasser vorgelegt und 49 g 2-Methylcyclopentanon und 68 g p-Anisaldehyd zugegeben. Es wurde unter Eigendruck und unter Rühren auf 120°C erhitzt und 3 Stunden unter Eigendruck (1,5 bar) nachgerührt. Nach Abkühlung auf Raumtemperatur wurde der ausgefallene Feststoff abgesaugt, mit Wasser neutral gewaschen und im Vakuumtrockenschrank bei 55°C getrocknet. So wurden 101,5 g 2-Methyl-5-(4-methoxybenzyliden)-cyclopentanon (gaschromatographisch rein) erhalten. Das entspricht einer Ausbeute von 94 % der Theorie.

### Beispiel 4

Es wurde verfahren wie in Beispiel 3, jedoch wurde anstelle von p-Anisaldehyd 87,5 g 2,4-Dichlorbenzaldehyd eingesetzt. Bei 120°C wies das Reaktionsgemisch dann einen Eigendruck von 1,2 bar auf. So wurden 116 g 2-Methyl-5-(2,4-dichlorbenzyliden)-cyclopentanon (93,7 %ig) erhalten. Das entspricht einer Ausbeute von 85 % der Theorie.

### Beispiel 5

In einem 0,3 l Edelstahl-Autoklaven wurde eine Lösung aus 1,1 g 85 %iger wäßriger Kalilauge in 100 ml Wasser vorgelegt und dann 25,2 g 2-Isopropylcyclopentanon und 28,1 g 4-Chlorbenzaldehyd hinzugefügt, unter Eigendruck auf 120°C erhitzt und 5 Stunden bei dieser Temperatur (Eigendruck 1 bar) nachgerührt. Nach der Abkühlung auf Raumtemperatur wurde die Suspension abgesaugt, das Produkt mit Wasser neutral gewaschen und anschließend im Vakuumtrockenschrank bei 55°C getrocknet. So wurden 46,8 g 2-Isopropyl-5-(4-chlorbenzyliden)-cyclopentanon (98,7 %ig) erhalten. Das entspricht einer Ausbeute von 93 % der Theorie.

### Beispiel 6

In einem 0,3 l Edelstahl-Autoklaven wurde eine Lösung aus 1,5 g 85 Gew.-%iger wäßriger Kalilauge in 135 ml Wasser vorgelegt und dann 47,5 g 2,4-Dichlorbenzaldehyd und 30,5 g 2-Methylcyclohexanon zugegeben und anschließend unter Eigendruck (bis 3,8 bar) auf 150°C erhitzt. Bei dieser Temperatur wurde 10 Stunden nachgerührt. Nach Abkühlung auf Raumtemperatur wurde die Suspension abgesaugt, mit Wasser neutral gewaschen und im Vakuumtrockenschrank bei 55°C getrocknet. So wurden 62,8 g 2-Methyl-6-(2,4-dichlorbenzyliden)-cyclohexanon (99,5 %ig) erhalten. Das entspricht einer Ausbeute von 86 % der Theorie.

## Patentansprüche

1. Verfahren zur Herstellung von Aryliden-substituierten Alkylcycloalkanonen der Formel (I) 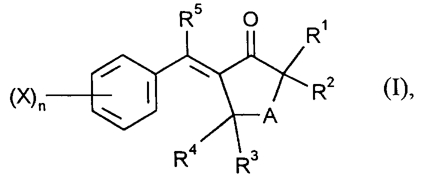 in der
A für gegebenenfalls substituiertes -(CH₂)-ₓ mit x = 1, 2 oder 3 steht,
R¹ und R² für gleiche oder verschiedene, gegebenenfalls substituierte C₁-C₄-Alkyl- oder C₃-C₇-Cycloalkylreste stehen oder R¹ und R² gemeinsam mit dem dazwischenliegenden C-Atom einen C₃-C₇-Cycloalkylrest bilden,
R³, R⁴ und R⁵ gleich oder verschieden sind und für Wasserstoff oder gegebenenfalls substituiertes C₁-C₄-Alkyl oder C₃-C₇-Cycloalkyl stehen, wobei R³ und R⁴ gemeinsam mit dem dazwischenliegenden C-Atom auch einen C₃-C₇-Cycloalkylrest bilden können,
X für Halogen, Cyano, Nitro, C₁-C₄-Alkyl, C₁-C₄-Halogenalkyl, C₁-C₄-Alkoxy oder C₁-C₄-Halogenalkoxy stehen, wobei beim Vorhandensein mehrerer X-Reste diese gleich oder verschieden sein können
und
n für null oder eine ganze Zahl von 1 bis 5 steht,
dadurch gekennzeichnet, daß man substituierte Cycloalkanone der Formel (II) 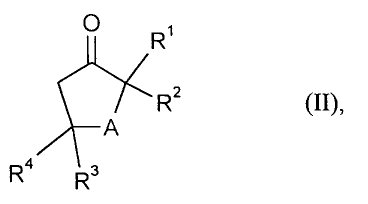 in der
die verwendeten Symbole die bei Formel (I) angegebene Bedeutung haben, mit aromatischen Carbonylverbindungen der Formel (III) 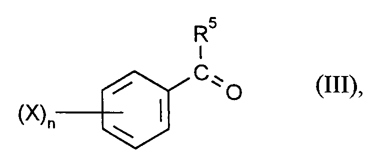 in der
die verwendeten Symbole die bei Formel (I) angegebene Bedeutung haben,
in Gegenwart eines basischen Katalysators und Wasser umsetzt.

2. Verfahren nach Anspruch 1, dadurch gekennzeichnet, daß in den Formeln
A eine oder mehrere, gleiche oder verschiedene Substituenten aus der Gruppe C₁-C₄-Alkyl und C₃-C₇-Cycloalkyl enthält, wobei C₁-C₄-Alkyl seinerseits gegebenenfalls durch Halogen oder C₁-C₄-Alkoxy und C₃-C₇-Cycloalkyl seinerseits gegebenenfalls durch Halogen, C₁-C₄-Alkyl oder C₁-C₄-Alkoxy substituiert sein kann,
soweit R¹ und R² für C₁-C₄-Alkyl stehen, dieses gegebenenfalls durch Halogen, C₁-C₄-Alkoxy, Halogen-C₁-C₄-alkoxy, C₂-C₄-Alkenyl, C₂-C₄-Alkinyl, Halogen-C₂-C₄-alkenyl oder Halogen-C₂-C₄-alkinyl substituiert ist und soweit R¹ und R² für C₃-C₇-Cycloalkyl stehen, dieses gegebenenfalls durch Halogen, C₁-C₄-Alkoxy oder C₁-C₄-Alkyl substituiert ist,
R³, R⁴ und R⁵ für Wasserstoff stehen,
X für Fluor, Chlor, Cyano oder C₁-C₄-Alkyl stehen und
n für 0, 1 oder 2 steht.

3. Verfahren nach Ansprüchen 1 und 2, dadurch gekennzeichnet, daß man als Verbindung der Formel (II) 2-Methylcyclopentanon, 2,2-Dimethylcyclopentanon, 2-Ethylcyclopentanon, 2,2-Diethylcyclopentanon, 2-Isopropylcyclopentanon, 2-Methylcyclohexanon oder 2-Methylcycloheptanon und als Verbindung der Formel (III) 4-Chlorbenzaldehyd, 4-Fluorbenzaldehyd, 4-Methoxybenzaldehyd, 4-Methylbenzaldehyd oder 2,4-Dichlorbenzaldehyd einsetzt.

4. Verfahren nach Ansprüchen 1 bis 3, dadurch gekennzeichnet, daß man als basische Katalysatoren Alkali- und Erdalkalihydroxide und/oder Alkali- und Erdalkalicarbonate und/oder Oxide dieser Elemente einsetzt, die bei den Reaktionsbedingungen Hydroxide oder Carbonate bilden.

5. Verfahren nach Ansprüchen 1 bis 4, dadurch gekennzeichnet, daß das Reaktionsmedium bis zu 20 Volumen-% organische Lösungsmittel enthält.

6. Verfahren nach Ansprüchen 1 bis 4, dadurch gekennzeichnet, daß als Reaktionsmedium Wasser ohne Zusätze anderer Lösungsmittel eingesetzt wird.

7. Verfahren nach Ansprüchen 1 bis 6, dadurch gekennzeichnet, daß man es bei Temperaturen von 20 bis 200°C und bei Drucken bis zu 10 bar durchführt.

8. Verfahren nach Ansprüchen 1 bis 7, dadurch gekennzeichnet, daß man pro Mol der Verbindung der Formel (III) 0,5 bis 2 Mol der Verbindung der Formel (II) einsetzt.

9. Verfahren nach Ansprüchen 1 bis 8, dadurch gekennzeichnet, daß man pro Mol der Verbindung der Formel (II) 0,05 bis 0,1 Äquivalente Katalysator einsetzt.

10. Verfahren nach Ansprüchen 1 bis 9, dadurch gekennzeichnet, daß man nach Durchführung des erfindungsgemäßen Verfahrens das vorliegende Reaktionsgemisch aufarbeitet, indem man es abkühlt, den vorliegenden Festkörper abfiltriert, mit Wasser wäscht und trocknet.
